(19) Europäisches Patentamt
European Patent Office
Office européen des brevets

(11) EP 1 069 879 B1

(12) EUROPEAN PATENT SPECIFICATION

(45) Date of publication and mention of the grant of the patent:
**16.06.2004 Bulletin 2004/25**

(21) Application number: **99910898.8**

(22) Date of filing: **01.03.1999**

(51) Int Cl.[7]: **A61F 13/15**, A61L 15/60

(86) International application number:
**PCT/SE1999/000284**

(87) International publication number:
**WO 1999/047093 (23.09.1999 Gazette 1999/38)**

# (54) ABSORBENT STRUCTURE AND PRODUCT BASED ON RAW MATERIALS HAVING A HIGH DEGREE OF RENEWABILITY

ABSORBIERENDE STRUKTUR UND PRODUKT AUS ROHMATERIALIEN MIT EINEM HOHEN ERNEUERUNGSGRAD

STRUCTURE ABSORBANTE ET ARTICLE A BASE DE MATIERE BRUTE ET A HAUT DEGRE DE RENOUVELLEMENT

(84) Designated Contracting States:
**AT BE CH CY DE DK ES FI FR GB GR IE IT LI LU MC NL PT SE**

(30) Priority: **16.03.1998 SE 9800846**

(43) Date of publication of application:
**24.01.2001 Bulletin 2001/04**

(73) Proprietor: **SCA Hygiene Products AB**
**405 03 Göteborg (SE)**

(72) Inventor: **LAGERSTEDT EIDRUP, Marie-Louise**
**S-427 37 Billdal (SE)**

(74) Representative: **Romare, Laila Anette**
**Albihns Göteborg AB**
**Box 142**
**401 22 Göteborg (SE)**

(56) References cited:
**EP-A1- 0 525 245**
**WO-A1-96/17681**
**WO-A1-98/10725**
**DE-A1- 1 961 680**
**FI-B- 100 852**
**US-A- 3 997 484**
**US-A- 5 506 277**
**US-A- 5 549 590**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 069 879 B1

## Description

TECHNICAL FIELD:

The invention relates to an absorbent structure which includes a highly absorbent polymer, i.e. a superabsorbent, which has been produced from one or more hydrophilic monomers by free radical copolymerization in the presence of starch and/or chemically modified starch. In addition, the invention relates to an absorbent product, such as a nappy, an incontinence shield, a sanitary towel or the like, which product includes the absorbent structure. The absorbent structure and the product are based on raw materials which have a high degree of renewability and which are in the main derived from non-fossil raw material sources.

BACKGROUND TO THE INVENTION:

In recent times, it has become ever more usual to use highly absorbent polymers, often termed superabsorbents, in absorbent structures for use, for example, in absorbent products which are intended for absorbing body fluids. Examples of such products are nappies, incontinence shields and sanitary towels. In this context, superabsorbents are understood to be polymers which are able to absorb several times their own weight of the fluid which is to be absorbed and which, during absorption, form fluid-containing gels.

The use of superabsorbents, or materials having superabsorbent properties, in absorbent structures present in absorbent products affords a number of advantages. As a result of using a superabsorbent, the volume of an absorbent product can be reduced substantially as compared with the volume of an absorbent product which only makes use of conventional absorptive materials such as, for example, cellulose-based fluff pulp, soft paper, nonwoven material and the like. This reduction in volume can be obtained without the absorption properties being impaired. Another advantage of superabsorbents is that they have a superior ability, as compared with many other absorptive materials, to retain absorbed fluid under pressure. Good ability to retain fluid is an advantage when an absorptive material is used in nappies, incontinence shields or sanitary towels, since the absorbed body fluid is retained in the product and is not pressed out of it when, for example, a user sits down.

In recent times, a great deal of effort has also been applied to developing different degradable plastic materials for use, for example, as surface or barrier materials in absorbent products. This should be evident, for example, from patent specifications EP 0 408 503 A2, WO 90/10671 and US 3,952,347, which all describe plastic materials which have been made at least partially degradable by adding starch. As a consequence, there are nowadays a number of different plastic materials which are at least partially degradable and which are available for use in absorbent products such as nappies, incontinence shields and sanitary towels. Several of these materials are based on combinations of synthetic polymers and biologically degradable starch. Even if utilizable surface and barrier materials now exist which are at least partially biologically degradable, it has been more difficult to find degradable replacement materials for the superabsorbents, which have so far generally been biologically non-degradable.

A feature of many of the superabsorbents which have been used to date which can be regarded as being a disadvantage is, therefore, that these superabsorbents are not biologically degradable and that they are not based on renewable raw materials either. The superabsorbents which have been used to date have commonly made use of synthetic polymers, such as acrylic acid, as the principal raw material.

For this reason, it has recently been proposed that use should be made of superabsorbents which are based on various renewable raw materials such as various polysaccharides and, in particular, starch. A feature of these previously known polysaccharide-based superabsorbents which can be regarded as being a disadvantage is the tendency of these superabsorbents to give rise to so-called gel blocking in association with fluid absorption into an absorptive body which contains such a superabsorbent. Specially constructed absorptive bodies having separate "pockets" for the superabsorbent, for example of the type described in US 5,433,715, have therefore been developed to prevent gel blocking from occurring.

However, since it is a relatively complicated matter to shape such specially constructed absorptive bodies, there exists a need for absorbent structures and products which do not require any special constructions and which are to a high degree based on renewable raw materials exhibiting high biological degradability.

The document US 5,549,590 discloses a high performance absorbent particle which contain a non-colloidal water resistant solid core encapsulated by a hydrogel forming polymer and wherein the solid core is, for example starch. A process for making such absorbent particles and an absorbent article comprising these particles are also disclosed in US 5,549,590. DE 196 19 680 discloses a method to produce a superabsorbent polymer with hydrophilic monomers in the presence of starch in bulk. As no solvent is used in the polymerization pprocess in DE 196 19 680 organophilic radical initiators must be used in the process.

ACCOUNT OF THE INVENTION:

A first object of the present invention is therefore that of providing an absorbent structure which is to a high degree based on renewable raw materials exhibiting high biological degradability and which nevertheless does not require any special shaping processes during its production.

This first object is achieved by the absorbent structure according to the invention, in accordance with the attached Patent Claim 1, including a superabsorbent which has been produced from one or more hydrophilic monomers by free radical copolymerization in the presence of starch and/or chemically modified starch, and by use having been made, during production of the superabsorbent, of a free radical initiator which forms three or more radical sites pér molecule, and by the absorbent structure including hydrophilic and/or hydrophobic fibres which, together with the superabsorbent, impart a hydrophilic character to the absorbent structure, and also by the superabsorbent constituting, in the dry state, between 10 and 75% of the dry weight of the absorbent structure.

In addition, a second object of the present invention is that of utilizing the absorbent structure according to the invention for providing an absorbent product having excellent absorption properties, which product is to a high degree based on renewable raw materials exhibiting high biological degradability.

This second object of the invention is achieved by the absorbent product according to the invention, in accordance with the attached Patent Claim 13, including an absorptive body which is enclosed by an enveloping material which is at least partially fluid-permeable, with the absorptive body including an absorbent structure which includes a superabsorbent which has been produced from one or more hydrophilic monomers by free radical copolymerization in the presence of starch and/or chemically modified starch, and by use having been made, during production of the superabsorbent, of a free radical initiator which forms three or more radical sites per molecule, and by the absorbent structure including hydrophilic and/or hydrophobic fibres which, together with the superabsorbent, impart a hydrophilic character to the absorbent structure, and also by the superabsorbent constituting, in the dry state, between 10 and 75% of the dry weight of the absorbent structure.

BRIEF DESCRIPTION OF THE FIGURES:

In that which follows, the invention will be described in greater detail with reference to the attached drawings in which

Fig. 1 shows a diagrammatic cross section of an absorbent structure according to an embodiment of the invention,

Fig. 2 shows a diagrammatic cross section of an absorbent structure according to a particularly preferred embodiment of the invention,

Fig. 3 shows a plane view of an absorbent product according to a preferred embodiment of the invention, as seen from the side which, during use, is intended to face a user, and

Fig. 4 shows a cross section along the line II-II through the absorbent product in Fig. 3.

DETAILED DESCRIPTION OF PREFERRED EMBODIMENTS:

Fig. 1 shows a diagrammatic cross section of an absorbent structure according to a first embodiment of the invention. In this case, structure 1 comprises a single layer 2, which consists of a mixture of fluff pulp, i.e. native cellulose fibres and a superabsorbent of a type which is specific for the invention. In Fig. 1, elongated cellulose fibres and granular superabsorbent particles have been indicated diagrammatically. In the first embodiment, the ratio in which the cellulose fibres and the superabsorbent are mixed is essentially constant through the whole of the absorbent structure 1. In this embodiment, the dry superabsorbent preferably constitutes between 20 and 30% by weight of the dry weight of the absorbent structure while the remainder is principally made up of cellulose fibres, which, together with the superabsorbent, impart a hydrophilic character, with a high level of ability to absorb and retain body fluids, to the absorbent structure 1.

Fig. 2 shows a second, particularly preferred embodiment of an absorbent structure 3 according to the invention. In this embodiment, structure 3 comprises several layers 4, 5, 6, of which at least a first layer 4 has a higher content of the superabsorbent which is specific for the invention than do the other layers 5, 6. In this connection, the superabsorbent preferably constitutes between 40 and 60% by weight of the dry weight of the first layer 4, with two opposite sides of the first layer 4 advantageously being in contact with the second layer, 5, and the third layer, 6, respectively, of the said several layers. In this way, the situation is achieved in which the first layer 4, having a higher content of superabsorbent, is covered on two opposite sides by layers 5, 6 having a lower content of superabsorbent. An embodiment of this nature has several advantages. One advantage is that the absorptive properties of the different layers 4, 5, 6 can be arranged to be different, depending on the intended application. Another advantage when using the absorbent structure in an absorbent product according to the invention is that the superabsorbent comes to be

located further away from the surface of the product, as a result of which the gel which is formed when the superabsorbent absorbs body fluid becomes less visible and perceptible. This can be an advantage, since it has been found that users can sometimes think that such a jelly-like substance, which is visible or felt from the outside of the product, can seem unpleasant or be regarded as being a sign of a defect in the quality of the absorbent product.

The specific superabsorbent which is utilized in the absorbent material structure and in the absorbent product according to the present invention can be said to be a hybrid superabsorbent which is based on a combination of a synthetic polymer and a natural polymer in the form of a starch and/or a starch derivative.

The superabsorbent is based on a hydrophilic polymeric composition which can be produced by the free radical (co)polymerization of the monomers which underlie the synthetic polymer in the presence of starch and/or starch derivatives. The polymerization is characterized by the use of a free radical initiator which can form triradicals or polyradicals. While the ratio of the weight of the synthetic polymer components and the starch or starch derivative components is between 90: 10 and 10:90, it is preferably between 60:40 and 30:70.

Monomers which are suitable for forming the basis for the synthetic polymer component and which may be mentioned are a number of polymerizable acids such as acrylic acid, methacrylic acid, caproic acid, vinylsulphonic acid, vinylphosphonic acid, maleic acid including its anhydride, fumaric acid, itaconic acid, 2-acrylamido-2-methylpropanesulphonic acid and its amides, hydroxyalkyl ester and esters and amides which contain amino or ammonium groups, and also water-soluble N-vinyl amide or diallylmethylammonium chloride.

The superabsorbent preferably contains hydrophilic monomers according to the general formula:

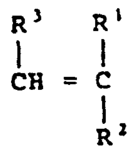

in which $R^1$ is hydrogen, methyl or ethyl; $R^2$ is $-COOR^4$ groups, sulphonyl groups, phosphonyl groups, phosphonyl groups which are esterified with $(C_1-C_4)$-alkanol or a group having the formula

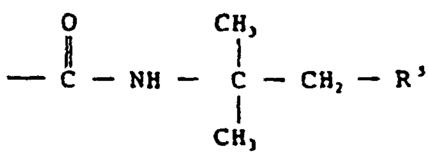

and $R^3$ is hydrogen, methyl, ethyl or carboxyl groups; $R^4$ is hydrogen, or amino- or hydroxy-$(C_1-C_4)$-alkyl; and $R^5$ is sulphonyl groups, phosphonyl groups or carboxyl groups.

Acrylic acid and methacrylic acid are particularly preferred hydrophilic monomers for use as the basis for the synthetic polymer component.

In principle, all naturally occurring starch types can be used as the basis for the natural polymer component, such as maize starch, wax-maize starch, potato starch, wheat starch, amylo-maize starch and tapioca starch. These can be used both in native and in pregelatinized form. In this context, pregelatinized maize starch and pregelatinized potato starch are particularly suitable.

A number of different chemically modified starches, or starch derivatives, can also be used for the natural polymer component, such as acid-catalytically, enzymically or thermally degraded starches, oxidized starches, starch ether such as allyl starch or hydroxyalkyl starches such as 2-hydroxyethyl starches, 2-hydroxypropyl starches or 2-hydroxy-3-trimethylammonionopropyl starches, or carboxyalkyl starches such as carboxymethyl starches, starch ester such as, for example, starch monocarboxylic ester such as starch formate, starch acetate, starch acrylate, starch methacrylate or starch benzoate, starch ester from dicarboxylic or polycarboxylic acids, such as starch succinate or starch maleate, starch carbamic ester (starch urethane), starch dithiocarbonic ester (starch xanthate), or starch ester of inorganic acids such as starch sulphate, starch nitrate or starch phosphate, starch ester ether such as, for example 2-hydroxyalkyl-starch acetate, or full acetals of starch, such as those which are formed, for example, by reacting starch with aliphatic or cyclic vinyl ether. In this context, carboxymethyl starch, starch succinate or starch maleate are particularly preferred.

In principle, all compounds which, with or without the influence of additional activators such as light, radiation, heat, ultrasound, reducing agents, etc., give rise to three or more radical sites per molecule can be used as free radical initiators. This implies that these free radical initiators contain three, four or more groups which can form free radicals.

While the radical sites can be formed simultaneously in this context, they are usually formed one after the other. Examples of suitable compounds are those which contain at least three hydroperoxide units, peroxide units or azo units. Suitable compounds of this nature are polyhydroperoxides which can be obtained by the anodic oxidation of polycarboxylic acids, in particular of polyacrylic acid and polymethacrylic acid in the presence of oxygen.

Peroxide units can be present, for example, as percarbonate, perketal or perester units. Examples of compounds of this nature are dioxetane compounds and tert-butyl peresters, such as, for example, methacrylate-tert-butyl peracrylate copolymers.

When producing the superabsorbent which is employed in accordance with the invention, it is preferred to use free radical initiators which contain peroxide units together with a reducing agent. Examples of suitable reducing agents in this context are $Fe^{2+}$, ascorbic acid, sulphinic acid, sulphite and formamidinesulphinic acids and their salts.

Suitable compounds which contain three or four azo units which can be mentioned by way of example are reaction products of azodicarboxylic acids with compounds which contain more than two oxirane functions, with a preferred azodicarboxylic acid being 4,4'-azobis(4-cyanovaleric acid), which forms suitable free radical initiators with, for example, polyglycerol-polyglycidyl ethers. Other suitable compounds within this group are products from the reaction of hydroxy- and amino-functional azo compounds with compounds which contain more than two oxirane groups. Examples of suitable compounds of this nature are 2,2'-azobis(N,N-dimethyleneisobutyramidine) or its corresponding dihydrochloride, 2,2'-azobis(2-amidinopropane) dihydrochloride, 2,2'-azobis(2-methyl-N-(1,1-bis(hydroxymethyl)-2-hydroxyethyl)propionamide), 2,2'-azobis(2-methyt-N-(1,1-bis(hydroxymethyl)ethyl)propionamide) or 2,2'-azobis (2-methyl-N-(2-hydroxyethyl)propionamide), which form suitable free radical initiators with, for example, polyglycerol-polyglycidyl ethers. Azobisnitriles with tri- or polyalcohols may also be mentioned. In this connection, products from the reaction of 2,2'-azobisisobutyronitrile with glycerol, trimethylolpropane, threitol, erythritol, pentaerythritol, arabitol, adonitol, xylitol, sorbitol, mannitol or dulcitol are particularly preferred.

When producing the superabsorbent in question, the previously mentioned free radical initiators may be used on their own or in arbitrary mixtures with each other. From 0.001 to 20% by weight, and preferably from 0.05 to 3.0% by weight, of the free radical initiator, calculated on the total quantity of monomer, is added in this context. While the molecular weight which is suitable for the free radical initiators employed can vary within wide limits, it is, in particular, within the range 100-10,000,000.

When producing the superabsorbent in question, it is particularly preferred to use free radical initiators whose free radical-forming functions possess different reactivities, i.e. which are activated by different mechanisms. Such initiators consequently contain both azo and peroxide or hydroperoxide functions, for example, which functions are activated one after the other in a predetermined manner and can consequently be used, for example, for producing block polymers.

In addition, it can be advantageous to use initiators whose radical-forming functions are located at different distances from each other in the molecule.

When producing the superabsorbent in question, it is furthermore possible to use suitable crosslinking agents, i.e. compounds which possess at least two double bonds and which can be polymerized into the lattice which is formed by the synthetic polymer component. Suitable crosslinking agents are, in particular, methylenebisacrylamide and methylenemethacrylamide, respectively, unsaturated mono- or polycarboxylic esters of polyols, such as diacrylate or triacrylate, e.g. butanediol or ethylene glycol diacrylate or methacrylate, trimethylolpropane triacrylate, as well as vinyl methacrylate and allyl compounds such as allyl (meth)acrylate, triallyl cyanurate, diallyl maleate, polyallyl esters, allyl ethers of polyols, such as, for example, pentaerythritol diallyl ether and pentaerythritol triallyl ether, tetraallyloxetane, triallylamine, tetraallylethylenediamine, allyl esters of phosphoric acid and also vinylphosphonic acid derivatives. From 0 to 20% by weight, and preferably from 0 to 3% by weight, of crosslinking agent, calculated on the total monomer fraction, is added in this context.

Using the abovementioned starting materials, the superabsorbent in question can be produced using previously known polymerization processes, for example by polymerization in aqueous phase in accordance with an inverse suspension polymerization process in the presence of the previously mentioned free radical initiator. Particular preference is given, in this context, to polymerization in a water-containing solution, i.e. so-called gel polymerization, with the dry matter content in the water-containing solution being between 15 and 60% by weight, the temperature being between 0 and 130°C, preferably between 10 and 100°C, and with it possible for the pressure to be atmospheric pressure or a pressure greater than this. In addition, the polymerization can also be carried out under a protective gas atmosphere, for example a nitrogen atmosphere. The basic polymerization process will not be described in more detail in this present document since it is well known to the skilled person in the field and lies outside the scope of the present invention.

The superabsorbent in question can also be crosslinked subsequently, in a manner known per se, in the water-containing gel phase and/or be crosslinked finally as ground and screened polymer particles. Compounds which contain at least two groups which can form covalent bonds with the carboxyl groups and/or hydroxyl groups of the polymer composition are suitable crosslinking agents for these purposes. Examples of suitable compounds which may

be mentioned are di- or polyglycidyl compounds, such as phosphonic acid diglycidyl ester, alkoxysilyl compounds, polyaziridine, polyamine, polyamidoamine and its reaction products with epichlorohydrin, di- or polyalcohols, divinyl sulphone, or di- or polyaldehydes such as glyoxal, for example. Phosphonic acid glycidyl ether and polyamidoamine-epichlorohydrin adducts are particularly preferred in this context.

After the polymerization has been completed, the quality properties of the polymerizate can be further improved by the water-containing polymerizate gel being heat-treated for a number of hours at 50-130°C, preferably at 70-100°C. After having been mechanically divided up using an apparatus which is suitable for the purpose, the gelatinous superabsorbent which has been obtained in this way can be dried by means of a previously known drying process in order to provide a superabsorbent in solid form which is suitable for use. A particularly preferred drying process in this context is cylinder drying, which enables the product to be dried under mild conditions.

After the superabsorbent has been obtained in dry, solid form, it is ready for use in an absorbent structure according to the present invention.

The superabsorbent which is employed in absorbent structures according to the invention imparts absorptive properties which are entirely satisfactory for the intended applications. Due to the fact that both the superabsorbent employed and other components in absorbent structures and products according to the invention are at least partially biologically degradable and at least partially based on renewable raw materials, the absorbent structures and products according to the invention offer great advantages as compared with conventional absorbent structures and products, which are often to a high degree based on synthetic raw materials which are non-degradable and often based on crude oil. These advantages are particularly evident in relation to disposable absorbent products, such as nappies, incontinence shields, sanitary towels or the like.

Absorbent structures and products according to the invention preferably comprise mixtures or combinations of the specific superabsorbent and various fibres such as fluff pulp consisting of cellulose, rayon, peat, cotton, hemp, flax or the like. In addition, various synthetic fibres, such as polyethene, polypropene, polyester, nylon, bicomponent fibres, split fibres, or the like, can be admixed. It is thus possible to use both hydrophobic and hydrophilic fibres, which can be of value with regard to ensuring different absorption properties in different layers in absorbent structures according to the invention.

In addition, the absorbent structure according to the invention can be bound or consolidated in different ways, for example by melting thermoplastic fibres which are included in the absorbent structure, or by adding a special binding agent. Furthermore, the absorbent structure according to the invention can have been subjected to additional processing such as pressing, embossing, calendering, needling, hydroentangling, mechanical softening or the like.

Figures 3 and 4 show an absorbent product 11 according to a preferred embodiment of the invention. In Fig. 3, the absorbent product 11 is shown as seen from the side which is intended, during use, to be facing the body of a user.

In the embodiment described, the absorbent product is a nappy 11 and includes a fluid-permeable surface material 12 on the side which is intended to be facing a wearer during use. In addition, the nappy 11 includes a fluid-impermeable barrier material 13 on the side which is intended to be facing away from the wearer during use. The fluid-permeable surface material 12 and the fluid-impermeable barrier material 13 together enclose an absorbent structure 3 according to the invention and can be said to constitute an enveloping material for the nappy 11.

In the embodiment described, the nappy 11 has an elongated form with wider front and rear sections 15, 16 and a narrower crotch section 17. The front section 15 is that part of the nappy 11 which, during use, is intended to be applied to the front side of a user while the rear section 16 is that part of the nappy 11 which, during use, is applied to the rear side of the user. In addition, the nappy 11 has two longitudinal, inwardly curved side edges 18, 19, a front edge 20 and a rear edge 21.

The nappy 11 is of the type which, during use, is fastened together in order thereby to enclose the lower part of the user's body in the manner of a pair of pants. For this purpose, a tape tab 22, 23 is arranged at, and projecting from, each side edge 18, 19 in the vicinity of the rear edge 21 of the nappy. The tape tabs 22, 23 are designed to interact with a fastening surface 24, which is arranged on the fluid-impermeable barrier material 13 on the front section 15 of the nappy 11. The fastening surface 24 preferably includes some form of reinforcement such as, for example, a further layer of plastic or a lining which has been applied to the fluid-impermeable barrier material 13. Alternative types of arrangements for fastening nappies together, such as buttons and buttonholes, hooks and eyes, press-studs, or the like, can, of course, also be used.

Furthermore, in the embodiment described, the nappy 11 is provided with longitudinal, elastic elements 25, 26, which are advantageously arranged along the side edges 18, 19. The elastic elements 25, 26 shape the nappy 11 and function during use as leg elastic. This means that the elastic elements 25, 26 hold the side edges 18, 19 of the nappy 11 in contact with the user's legs during use and prevent the formation of gaps between the nappy and the user's body, which gaps could otherwise lead to fluid leaking out from the nappy 11. It is also, of course, possible to conceive of embodiments of the product according to the invention in which the elastic elements are arranged in another manner which is suitable for the application.

In a corresponding manner, elastic elements 27, 28 are arranged along the front edge 20 and the rear edge

21, with the aim of creating elastic seals around the user's waist.

The absorbent structure 3 according to the invention can be said to constitute an absorptive core in the described nappy 11, as is evident from the attached Fig. 4. In the embodiment described, the previously mentioned second layer 5, immediately inside the fluid-permeable surface material 12, can be said to constitute a fluid reception layer whose task is rapidly to transport fluid into the previously mentioned first layer 4, which has a higher content of superabsorbent than the other layers 5, 6. In this context, the task of the first layer 4 is to absorb, store and bind fluid. In the embodiment described, the third layer 6 of the absorbent structure 3 functions as a spreading layer, whose task is to transport fluid onwards to unused parts of the first layer 4, in order to permit renewed absorption in those parts of the first layer 4 from which fluid has been transported away with the aid of the third layer 6. Such an effect can be achieved in a manner which is well known to the skilled person, for example by choosing the raw fibre material and content of superabsorbent in the different layers so as to thereby influence, for example, the capillary forces and hydrophilicity in the different layers in a desired manner.

It is also possible to conceive of embodiments of the invention in which the fluid reception layer and the spreading layer are not included in the absorbent structure 3 but are instead provided as separate layers of material. A suitable separate fluid reception layer in such an embodiment advantageously consists of a relatively thick, bulky fibre material which exhibits high resilience both in the dry and wet states in order to avoid collapse in the wet state. Examples of suitable fibre materials in this context are nonwoven materials which are based on stiffened cellulose fibres and/or synthetic fibres such as polyethene fibres, polypropene fibres, polyester fibres or the like.

In embodiments of the product according to the invention which possess separate fluid reception and spreading layers, the absorbent structure is advantageously of the type which has previously been described with reference to the attached Fig. 1, i.e. comprising a single layer with essentially the same mixing ratio between superabsorbent and fibres. The spreading layer in such an embodiment advantageously consists of a separate, powerfully compressed layer of hydrophilic fibres which provide small capillaries and, as a result, strong capillary forces and good spreading ability. Materials which have been found to be particularly suitable for this application, and which may be mentioned, are those which are described in WO 94/10953 and WO 94/10956.

The invention can be varied in many ways within the scope of the attached patent claims. As has already become evident, it is possible to conceive both of embodiments of absorbent products according to the invention in which the absorbent structure according to the invention in principle consists of the whole absorptive body and also of embodiments in which the absorbent structure is combined with separate fluid reception or spreading layers consisting of materials which are suitable for these purposes.

EXAMPLES:

With a view to illustrating the invention, absorptive bodies were manufactured in a standardized manner using a pilot-scale shaping device, a so-called sheet former, in accordance with a technique which is well known to the skilled person. The resulting absorptive bodies were then evaluated using test methods which have been found to be suitable for absorbent structures which are intended for use in absorbent products for absorbing body fluids.
The absorptive bodies were manufactured by fluff pulp consisting of bleached chemical softwood pulp being mixed with different superabsorbents in a device intended for the purpose and subsequently being shaped in the sheet former into an absorptive core having one or more layers, after which the absorptive core was provided with surface material in order to form an absorptive body for evaluating absorptive properties.

In the experimental series, absorptive bodies were manufactured which had four different absorptive cores in accordance with the following four experimental series:

<u>MIX REF:</u>

a layer of fluff pulp containing a homogeneous admixture of 25% by weight of conventional acrylic acid-based superabsorbent,

<u>MIX INV:</u>

a layer of fluff pulp containing a homogeneous admixture of 25% by weight of the superabsorbent, containing 30% by weight of starch, which was employed in accordance with the invention,

<u>LAYER REF:</u>

two identical outer layers of 100% by weight of fluff pulp with an intervening, inner layer containing a homogeneous admixture of 50% by weight of conventional acrylic acid-based superabsorbent, with the content of

superabsorbent, calculated on the weight of the whole of the absorptive core, being 33% by weight, and

LAYER INV:

two identical outer layers of 100% by weight fluff pulp with an intervening, inner layer containing a homogeneous admixture of 50% by weight of the superabsorbent, containing 30% by weight of starch, which was employed in accordance with the invention, with the content of superabsorbent, calculated on the weight of the whole of the absorptive core, being 33% by weight.

The absorptive bodies were provided with conventional surface materials, namely a fluid-impermeable polyethene film on the outside of the absorptive core on that side which, in an absorbent product, should, during use, be facing a user, while the opposite side of the absorptive core, i.e. the front side, was provided with a thin, hydrophobic but fluid-permeable, nonwoven.

The absorptive bodies having the four different types of absorptive cores were then evaluated with regard to absorptive properties which were important for the application using the following test methods:

Admission time and rewetting

The test method is principally intended for evaluating different superabsorbents in an absorptive body. In principle, the method comprises measuring the fluid-holding ability of the absorptive body on loading and also measuring absorption time. The test method is carried out in the following manner:

1. Absorptive bodies having the x, y dimensions 28 x 10 cm are manufactured in the previously mentioned sheet former, with the area weight being approx. 1000 $g/m^2$, of which the pulp area weight constitutes approx. 750 $g/m^2$. The pulp bulk is adjusted so that it is finally approximately 8 $cm^3/g$. As has previously been made clear, the core of the absorptive bodies additionally contains the desired content of superabsorbent.
2. Before being tested, the absorptive bodies are conditioned at 23°C and 50% RH for 24 ± 2 h.
3. Before being tested, the absorptive bodies are weighed, with the weight of the surface material being subtracted before the area weight of the absorptive core is calculated. The absorptive bodies are marked at the intended point of wetting.
4. The thickness of the absorptive bodies is measured in a thickness gauge which is suitable for the purpose, and the exact bulk is calculated. If the bulk is too high, the absorptive body is pressed in a pressing device which is suitable for the purpose. Absorptive bodies whose bulk is too low are discarded.
5. A volume of 60 ml of test liquid is measured out. The test liquid employed consists of so-called synthetic urine, i.e. a salt/water solution which is adapted to resemble human urine.
6. A Plexiglas tube having an internal diameter of 23 mm is placed on top of the wetting point mark on the absorptive body.
7. The test liquid is added through the Plexiglas tube, such that a liquid column of 20 mm is maintained during the addition. The time (t1) for the whole of the liquid volume to be absorbed is determined using a timer which is accurate to 0.01s.
8. The dry weight of a sheaf of 15 pieces of filter paper is determined to an accuracy of 0.01 g.
9. 10 minutes after the absorption has begun, the sheaf of filter papers + a loading weight of 2550 g are placed on top of the wetted absorptive body.
10. After the loading has been applied on top of the absorptive body and the intervening filter papers for 15 seconds, the loading weight and the sheaf of filter papers are removed, with the wet weight of the filter papers being determined to an accuracy of 0.01 g. After this, the rewetting (r1) is calculated as a difference between the wet and dry weights of the filter papers.
11. A further 60 ml of test liquid + compensation for the previously calculated rewetting (r1) are then added.
12. By repeating items 7-11 above twice, additional absorption times (t2 and t3) and additional rewettings (r2 and r3) are determined in association with repeated wettings. In conclusion, t1, t2 and t3 are recorded to an accuracy of 1s, and r1, r2 and r3 are recorded to an accuracy of 0.1 g. TABLE 1 gives the values of t1, t2 and t3, and r1, r2 and r3, for the four different absorptive bodies in the example.

TABLE 1

| Experiment | Admission time, sec. | | | Rewetting, g | | |
|---|---|---|---|---|---|---|
| | t1 | t2 | t3 | r1 | r2 | r3 |
| MIX REF | 14 | 11 | 15 | 0.4 | 7.3 | 14.1 |
| MIX INV | 13 | 18 | 28 | 1.1 | 11.3 | 20.6 |
| LAYER REF | 33 | 42 | 50 | 0.6 | 7.0 | 19.5 |
| LAYER INV | 31 | 38 | 48 | 1.5 | 9.1 | 25.0 |

As can be seen from the test results obtained, the differences in the admission time t1 between MIX REF and MIX INV and between LAYER REF and LAYER INV, respectively, are relatively small. It thus appears as if the absorptive bodies according to the invention (INV) exhibit an initial admission time t1 which is of the same order as that of the corresponding conventional absorptive body (REF).

With regard to t2 and t3 for the two absorptive bodies containing a homogeneous mixture of fluff pulp and superabsorbent (MIX REF and MIX INV), it appears as if the absorptive body according to the invention (MIX INV) exhibits somewhat longer admission times (t3 and t3) than does the corresponding conventional absorptive body (MIX REF).

However, the test results for the two layered absorptive bodies (LAYER REF and LAYER INV) show that it is possible to produce absorptive bodies according to the invention (LAYER INV) which have admission times (t2, t3) in association with repeated wetting which are as good as those for a corresponding conventional absorptive body (LAYER REF).

All the measurements of rewetting (r1, r2 and r3) give higher values for the absorptive bodies in accordance with the invention (MIX INV and LAYER INV) than for the corresponding conventional absorptive bodies (MIX REF and LAYER REF).

However, the test results show that all the absorptive bodies produced in the experiments possess sufficiently good values with regard to both admission time and rewetting to be able to function extremely well in an absorbent product, such as a nappy, an incontinence shield or a sanitary towel.

Liquid spreading

Another important property of absorptive bodies is the ability to spread liquid. A measurement of this property which is significant for the application can be obtained by a test method as described below:

1. The absorptive bodies which are to be evaluated are conditioned as described above and provided with a mark which indicates a distance of 11 cm from one of the short ends.
2. A liquid container is placed beside a balance which is accurate to 0.1 g, with both the liquid container and the balance being carefully adjusted to the horizontal plane using a spirit level.
3. An oblong Plexiglas plate is arranged, using support devices which are suitable for the purpose, on top of the balance such that the Plexiglas plate is inclined at 30° to the horizontal plane and such that one of the short ends of the Plexiglas plate projects down into the liquid container. At the same time, the inclined Plexiglas plate is not allowed to touch the edge of the liquid container.
4. Test liquid, i.e. synthetic urine, is added to the container until 2 cm of the length of the inclined Plexiglas plate project below the surface of the liquid.
5. The absorptive body which is to be evaluated is weighed to an accuracy of 0. 1 g in order to give its dry weight ($m_1$).
6. The absorptive body is placed on the inclined Plexiglas plate with the fluid-permeable side upwards and in such a position that it does not come in contact with the test liquid in the liquid container; the balance is then tared.
7. After that, the absorptive body is fixed with a clamp in a predetermined position, using the previously mentioned 11 cm mark and a corresponding mark on the Plexiglas plate, on top of the inclined Plexiglas plate, without the absorptive body being allowed to come into contact with a test liquid.
8. The absorptive body is now allowed to fall down on top of the inclined Plexiglas plate and, in so doing, one of its short ends comes to dip down into the test liquid in the liquid container.
9. Using a computer, the balance reading is recorded continuously for 60 minutes while the level of the test liquid in the liquid container is kept constant, after which the measurement is discontinued and the absorptive body is removed from its position on top of the inclined Plexiglas plate.
10. The wetting length of the wetted region in the longitudinal direction of the absorptive body is measured in cm.
11. The liquid-spreading ability of the absorptive body after 5 and 60 minutes of measurement, respectively, is

calculated in the unit g/g in accordance with the formula;

$$\text{liquid spreading} = m_2/m_1,$$

in which $m_1$ is the dry weight of the absorptive body while $m_2$ is the weight read off the balance at the respective time point.

TABLE 2 below gives the results obtained from the measurement of liquid spreading carried out on the four previously described absorptive bodies.

TABLE 2

| Experiment | Liquid spreading, g/g | | Wetting length 60 min, cm |
|---|---|---|---|
| | 5 min | 60 min | |
| MIX REF | 9.3 | 14.2 | 28 |
| MIX INV | 8.2 | 11.1 | 26.7 |
| LAYER REF | 8.9 | 15.9 | 28 |
| LAYER INV | 8.1 | 14.4 | 28 |

As can be seen from TABLE 2 above, the absorptive bodies according to the invention (MIX INV and LAYER INV, respectively) each exhibited a somewhat lower liquid-spreading ability than did the corresponding conventional absorptive body (MIX REF and LAYER REF, respectively).

In summary, absorbent structures according to the invention offer fully satisfactory absorptive properties for use in absorbent products, without any specially adapted shaping processes and the like being required during manufacture. In addition, the invention offers further advantages such as the fact that absorbent structures and products according to the invention are to a high degree based on renewable raw materials exhibiting high biological degradability.

In the foregoing, the invention has been described with reference to attached figures, preferred embodiments and examples. However, the invention should in no way be regarded as being limited to that which has emerged in this context; on the contrary, its scope is defined by the subsequent patent claims.

Thus, the content of starch or starch derivatives in the superabsorbent employed by the invention can, for example, be varied within wide limits. Bearing in mind the fact that one of the main aims of the invention is to provide absorbent structures and products which are based on raw materials having high renewability, embodiments are particularly advantageous in which the dry superabsorbent contains more than 30% by weight of starch and/or chemically modified starch and in which, at the same time, the dry superabsorbent constitutes between 15 and 65% of the dry weight of the absorbent structure.

In particularly preferred embodiments of absorbent products according to the invention, the enveloping material is also at least partially biologically degradable, with the aim of improving compostibility, and at least partially based on raw materials exhibiting high renewability. In this connection, the content of biologically degradable material, preferably starch, is preferably greater than 10% by weight of the weight of the enveloping material.

## Claims

1. Absorbent structure which contains a superabsorbent which has been produced from one or more hydrophilic monomers by free radical copolymerization in the presence of starch and/or chemically modified starch,
**characterized in that** use has been made, during production of the superabsorbent, of a free radical initiator which forms three or more radical sites per molecule, and that the free radical copolymerization has been performed in aqueous phase, and **in that** the absorbent structure (1, 3) includes hydrophilic and/or hydrophobic fibres which, together with a superabsorbent, impart a hydrophilic character to the absorbent structure, and **in that**, in the dry state, the superabsorbent is a hybrid superabsorbent which is based on a combination of a synthetic polymer and a natural polymer in the form of a starch and/or a starch derivative and the superabsorbent constitutes between 10 and 75% of the dry weight of the absorbent structure.

2. Absorbent structure according to Claim 1,
**characterized in that** the dry superabsorbent contains more than 30% by weight of starch and/or chemically

modified starch, and **in that**, in the dry state, the superabsorbent constitutes between 15 and 65% by weight of the dry weight of the absorbent structure (1, 3).

3. Absorbent structure according to Claim 1 or 2,
**characterized in that** the absorbent structure (1) comprises a single layer (2) exhibiting essentially a constant mixing ratio between the said fibres and the said superabsorbent, and **in that**, in the dry state, the superabsorbent constitutes between 20 and 30% by weight of the dry weight of the absorbent structure (1).

4. Absorbent structure according to Claim 1 or 2,
**characterized in that** the absorbent structure (3) includes several layers (4, 5, 6), of which at least a first layer (4) has a higher content of superabsorbent than the other layers (5, 6), and **in that**, in the dry state, the superabsorbent [in this context] constitutes between 40 and 60% by weight of the dry weight of the first layer (4).

5. Absorbent structure according to Claim 4,
**characterized in that** the first layer (4) is in contact, on two opposite sides, with layers (5, 6) of the said several layers having a lower content of superabsorbent than in the said first layer (4).

6. Absorbent structure according to any one of the preceding claims,
**characterized in that** the superabsorbent includes hydrophilic monomers according to the general formula:

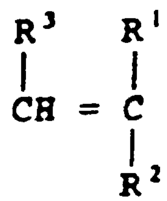

in which $R^1$ is hydrogen, methyl or ethyl; $R^2$ is $-COOR^4$ groups, sulphonyl groups, phosphonyl groups, phosphonyl groups which are esterified with $(C_1-C_4)$-alkanol or a group having the formula

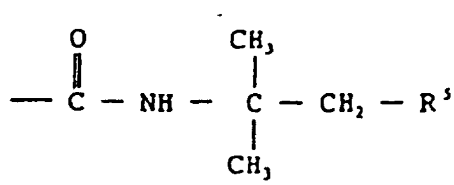

and $R^3$ is hydrogen, methyl, ethyl or carboxyl groups; $R^4$ is hydrogen, or amino or hydroxy-$(C_1-C_4)$-alkyl; and $R^5$ is sulphonyl groups, phosphonyl groups or carboxyl groups.

7. Absorbent structure according to any one of the preceding claims, **characterized in that** the starch which is included in the superabsorbent is native or pregelatinized maize starch, native or pregelatinized wax maize starch, native or pregelatinized potato starch, native or pregelatinized wheat starch, native or pregelatinized amylo-maize starch or native or pregelatinized tapioca starch, and/or **in that** the chemically modified starch which is included in the superabsorbent is acid-catalytically, enzymically or thermally degraded starch, oxidized starch, starch ether such as allyl starch or hydroxyalkyl starch such as 2-hydroxyethyl starch, 2-hydroxypropyl starch or 2-hydroxy-3-trimethylammonionopropyl starch, or carboxyalkyl starch such as carboxymethyl starch, starch ester such as starch formate, starch acetate, starch acrylate, starch methacrylate or starch benzoate, starch ester such as starch succinate or starch maleate, starch carbamic ester (starch urethane), starch dithiocarbonic ester (starch xanthate), or starch ester of inorganic acids such as starch sulphate, starch nitrate or starch phosphate, starch ester ether such as 2-hydroxyalkyl-starch acetate, or full acetals of starch, such as reaction products from starch with aliphatic or cyclic vinyl ether.

8. Absorbent structure according to any one of the preceding claims, **characterized in that** the starch which is included in the superabsorbent is pregelatinized maize starch or pregelatinized potato starch, and/or **in that** the

chemically modified starch which is included in the superabsorbent is carboxymethyl starch, starch succinate or starch maleate.

**9.** Absorbent structure according to any one of the preceding claims, **characterized in that** the free radical initiator employed when producing the superabsorbent is a compound which contains at least three hydroperoxide units, peroxide units or azo units.

**10.** Absorbent structure according to any one of the preceding claims, **characterized in that** the free radical initiator employed when producing the superabsorbent is a reaction product from azobisbutyronitrile and trimethylolpropane.

**11.** Absorbent structure according to any one of the preceding claims, **characterized in that** the free radical initiator employed when producing the superabsorbent is a polyhydroperoxide which has been obtained by the anodic oxidation of a polycarboxylic acid in the presence of oxygen.

**12.** Absorbent structure according to any one of the preceding claims, **characterized in that** the superabsorbent has been produced by polymerizing a solution containing between 15 and 60% by weight of one or more hydrophilic monomers, in the presence of starch and/or chemically modified starch, by means of a gel polymerization process in the presence of a free radical initiator which is able to form triradicals or polyradicals.

**13.** Absorbent product (11), such as a nappy, an incontinence shield or a sanitary towel, which includes an absorptive body enclosed by an enveloping material which is at least partially fluid-permeable, **characterized in that** said absorptive body includes an absorbent structure according to any one of the Claims 1 to 12.

**14.** Absorbent product according to Claim 13, in which the enveloping material includes a fluid-permeable surface material (12) on the side of the absorbent structure (3) which is intended to be facing a wearer during use and a fluid-impermeable barrier material (13) on the side of the absorbent structure (3) which is intended to be facing away from a wearer during use, **characterized in that** the fluid-permeable surface material (12) and/or barrier material (13) includes one or more biologically degradable materials having a high degree of renewability in an admixture which is greater than about 10% by weight of the total weight of the enveloping material.

**15.** Absorbent product according to Claim 14, **characterized in that** starch is included in the biologically degradable material.

**Patentansprüche**

**1.** Absorbierende Struktur, die einen Superabsorber enthält, der aus einem oder mehreren hydrophilen Monomeren durch freie radikalische Copolymerisation in Gegenwart von Stärke und/oder chemisch modifizierter Stärke hergestellt wurde, **dadurch gekennzeichnet, dass** während der Herstellung des Superabsorbers von einem freien Radikalstarter Gebrauch gemacht wurde, der drei oder mehr radikalische Stellen pro Molekül bildet, und dass die freie radikalische Copolymerisation in wässriger Phase durchgeführt wurde, und dass die absorbierende Struktur (1, 3) hydrophile und/oder hydrophobe Fasern beinhaltet, die zusammen mit einem Superabsorber der absorbierenden Struktur einen hydrophilen Charakter verleihen und dass im trockenen Zustand der Superabsorber ein Hybrid-Superabsorber ist, der auf einer Kombination eines synthetischen Polymers und eines natürlichen Polymers in Form von Stärke und/oder einem Stärkederivat beruht, und der Superabsorber zwischen 10 und 75 % des Trockengewichts der absorbierenden Struktur ausmacht.

**2.** Absorbierende Struktur gemäss Anspruch 1, **dadurch gekennzeichnet, dass** der trockene Superabsorber mehr als 30 Gew.% Stärke und/oder chemisch modifizierte Stärke enthält, und dass in trockenem Zustand der Superabsorber zwischen 15 und 65 Gew.% des Trockengewichts der absorbierenden Struktur (1, 3) ausmacht.

**3.** Absorbierende Struktur gemäss Anspruch 1 oder 2, **dadurch gekennzeichnet, dass** die absorbierende Struktur (1) eine einzelne Schicht (2) umfasst, die im wesentlichen ein konstantes Mischverhältnis zwischen den Fasern und dem Superabsorber aufweist, und dass in trockenem Zustand der Superabsorber zwischen 20 und 30 Gew. % des Trockengewichts der absorbierenden Struktur (1) ausmacht.

**4.** Absorbierende Struktur gemäss Anspruch 1 oder 2, **dadurch gekennzeichnet, dass** die absorbierende Struktur

(3) verschiedene Schichten (4, 5, 6) beinhaltet, von denen mindestens eine erste Schicht (4) einen höheren Gehalt an Superabsorber aufweist als die anderen Schichten (5, 6), und dass in trockenem Zustand der Superabsorber [in diesem Zusammenhang] zwischen 40 und 60 Gew.% des Trockengewichts der ersten Schicht (4) ausmacht.

**5.** Absorbierende Struktur gemäss Anspruch 4, **dadurch gekennzeichnet, dass** sich die erste Schicht (4) auf zwei gegenüberliegenden Seiten in Kontakt mit Schichten (5, 6) der besagten mehreren Schichten befindet, die einen geringeren Gehalt an Superabsorber aufweisen als die erste Schicht (4).

**6.** Absorbierende Struktur gemäss einem der vorangehenden Ansprüche, **dadurch gekennzeichnet, dass** der Superabsorber hydrophile Monomere entsprechend der folgenden allgemeinen Formel beinhaltet:

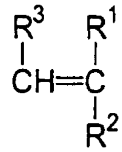

worin $R^1$ Wasserstoff, Methyl oder Ethyl ist; $R^2$ -$COOR^4$-Gruppen, Sulfonylgruppen, Phosphonylgruppen, Phosphonylgruppen, die mit einem ($C_{1-4}$)-Alkanol verestert sind, oder eine Gruppe der folgenden Formel ist:

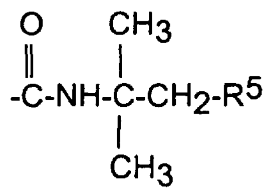

und $R^3$ ist Wasserstoff, Methyl, Ethyl oder Carboxylgruppen; $R^4$ ist Wasserstoff oder Amino oder Hydroxy-($C_{1-4}$)-alkyl; und $R^5$ ist Sulfonylgruppen, Phosphonylgruppen oder Carboxylgruppen.

**7.** Absorbierende Struktur gemäss einem der vorangehenden Ansprüche, **dadurch gekennzeichnet, dass** die im Superabsorber enthaltene Stärke natürliche oder vorgelatinierte Maisstärke, natürliche oder vorgelatinierte Wachsmaisstärke, natürliche oder vorgelatinierte Kartoffelstärke, natürliche oder vorgelatinierte Weizenstärke, natürliche oder vorgelatinierte Amylomaisstärke oder natürliche oder vorgelatinierte Tapiocastärke ist und/oder dass die im Superabsorber enthaltene, chemisch modifizierte Stärke Säure-katalytisch, enzymatisch oder thermisch abgebaute Stärke, oxidierte Stärke, Stärkeether, wie Allylstärke, oder Hydroxyalkylstärke, wie 2-Hydroxyethylstärke, 2-Hydroxypropylstärke oder 2-Hydroxy-3-trimethyl-ammonionopropylstärke, oder Carboxyalkylstärke, wie Carboxymethylstärke, Stärkeester, wie Stärkeformiat, Stärkeacetat, Stärkeacrylat, Stärkemethacrylat oder Stärkebenzoat, Stärkeester, wie Stärkesuccinat oder Stärkemaleat, Stärkecarbaminsäureester (Stärkeurethan), Stärkedithiokohlensäureester (Stärkexanthat) oder Stärkeester von anorganischen Säuren, wie Stärkesulfat, Stärkenitrat oder Stärkephosphat, Stärkeesterether, wie 2-Hydroxyalkyl-stärkeacetat, oder Vollacetale der Stärke, wie Reaktionsprodukte aus Stärke mit aliphatischen oder cyclischen Vinylethern ist.

**8.** Absorbierende Struktur gemäss einem der vorangehenden Ansprüche, **dadurch gekennzeichnet, dass** die im Superabsorber enthaltene Stärke vorgelatinierte Maisstärke oder vorgelatinierte Kartoffelstärke ist und/oder dass die im Superabsorber enthaltene, chemisch modifizierte Stärke Carboxymethylstärke, Stärkesuccinat oder Stärkemaleat ist.

**9.** Absorbierende Struktur gemäss einem der vorangehenden Ansprüche, **dadurch gekennzeichnet, dass** der bei der Herstellung des Superabsorbers eingesetzte freie Radikalstarter eine Verbindung ist, die mindestens drei Hydroperoxideinheiten, Peroxideinheiten oder Azoeinheiten enthält.

10. Absorbierende Struktur gemäss einem der vorangehenden Ansprüche, **dadurch gekennzeichnet, dass** der bei der Herstellung des Superabsorbers eingesetzte freie Radikalstarter ein Reaktionsprodukt aus Azobisbutyronitril und Trimethylolpropan ist.

11. Absorbierende Struktur gemäss einem der vorangehenden Ansprüche, **dadurch gekennzeichnet, dass** der bei der Herstellung des Superabsorbers eingesetzte freie Radikalstarter ein Polyhydroperoxid ist, das man durch anodische Oxidation einer Polycarbonsäure in Gegenwart von Sauerstoff erhalten hat.

12. Absorbierende Struktur gemäss einem der vorangehenden Ansprüche, **dadurch gekennzeichnet, dass** der Superabsorber dadurch erhalten wurde, dass eine Lösung, die zwischen 15 und 60 Gew.% eines oder mehrerer hydrophiler Monomere enthält, in Gegenwart von Stärke und/oder chemisch modifizierter Stärke mittels eines Gelpolymerisationsverfahrens in Gegenwart eines freien Radikalstarters polymerisiert wurde, der in der Lage ist, Triradikale oder Polyradikale zu bilden.

13. Absorbierendes Produkt (11), wie eine Windel, ein Inkontinenzschutz oder eine Damenbinde, welches einen absorbierenden Körper beinhaltet, der von einem einhüllenden Material umschlossen wird, welches zumindest teilweise flüssigkeitsdurchlässig ist, **dadurch gekennzeichnet, dass** der absorbierende Körper eine absorbierende Struktur gemäss einem der Ansprüche 1 bis 12 aufweist.

14. Absorbierendes Produkt gemäss Anspruch 13, worin das einhüllende Material ein flüssigkeitsdurchlässiges Oberflächenmaterial (12) auf der Seite der absorbierenden Struktur (3), welche dem Träger während des Gebrauchs zugewandt sein soll, und ein flüssigkeitsundurchlässiges Barrierematerial (13) auf der Seite der absorbierenden Struktur (3) enthält, welche dem Träger während des Gebrauchs abgewandt sein soll **dadurch gekennzeichnet, dass** das flüssigkeitsdurchlässige Oberflächenmaterial (12) und/oder das Barrierematerial (13) ein oder mehrere biologisch abbaubare Materialien mit einem hohen Erneuerungsgrad in einer Beimischung enthält, die grösser als etwa 10 Gew.% des Gesamtgewichts des einhüllenden Materials ist.

15. Absorbierendes Produkt gemäss Anspruch 14, **dadurch gekennzeichnet, dass** Stärke im biologisch abbaubaren Material enthalten ist.

**Revendications**

1. Structure absorbante qui contient un superabsorbant qui a été produit à partir d'un ou plusieurs monomères hydrophiles par copolymérisation radicalaire en présence d'amidon et/ou d'amidon chimiquement modifié, structure **caractérisée en ce qu'**on a utilisé, lors de la production du superabsorbant, un amorceur radicalaire qui forme trois ou plus de trois sites radicalaires par molécule, et on a réalisé la copolymérisation radicalaire en phase aqueuse, et **en ce que** la structure absorbante (1,3) comprend des fibres hydrophiles et/ou des fibres hydrophobes qui, associées au superabsorbant, communiquent un caractère hydrophile à la structure absorbante, et **en ce que**, à l'état sec, le superabsorbant est un superabsorbant hybride qui est à base d'une combinaison d'un polymère synthétique et d'un polymère naturel sous la forme d'amidon et/ou d'un dérivé de l'amidon, et le superabsorbant constitue de 10 à 75 % du poids à sec de la structure absorbante.

2. Structure absorbante selon la revendication 1, **caractérisée en ce que** le superabsorbant sec contient plus de 30 % en poids d'amidon et/ou d'amidon chimiquement modifié, et **en ce que**, à l'état sec, le superabsorbant constitue de 15 à 65 % en poids du poids à sec de la structure absorbante (1,3).

3. Structure absorbante selon la revendication 1 ou 2, **caractérisée en ce que** la structure absorbante (1) comprend une couche unique (2) présentant pratiquement un rapport de mélange constant entre lesdites fibres et ledit superabsorbant, et **en ce que**, à l'état sec, le superabsorbant constitue de 20 à 30 % en poids du poids à sec de la structure absorbante (1).

4. Structure absorbante selon la revendication 1 ou 2, **caractérisée en ce que** la structure absorbante (3) comprend plusieurs couches (4,5,6) dont au moins une première couche (4) a une teneur en superabsorbant plus élevée que celle des autres couches (5,6), et **en ce que**, à l'état sec, le superabsorbant constitue dans ce contexte 40 à 60 % en poids du poids à sec de la première couche (4).

5. Structure absorbante selon la revendication 4, **caractérisée en ce que** la première couche (4) est en contact, sur

les deux côtés opposés, avec des couches (5,6) desdites plusieurs couches ayant une teneur en superabsorbant inférieure à celle de ladite première couche (4).

**6.** Structure absorbante selon l'une quelconque des revendications précédentes, **caractérisée en ce que** le superabsorbant comprend des monomères hydrophiles de formule générale :

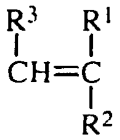

dans laquelle $R^1$ représente un atome d'hydrogène ou un groupe méthyle ou éthyle, $R^2$ représente un groupe $-COOR^4$, un groupe sulfonique, un groupe phosphonique, un groupe phosphonique estérifié par un alcanol en $C_1$ à $C_4$, ou un groupe de formule

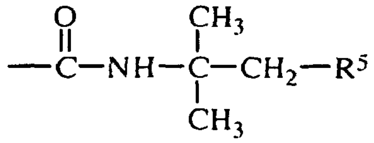

$R^3$ représente un atome d'hydrogène ou un groupe méthyle, éthyle ou carboxyle, $R^4$ représente un atome d'hydrogène ou un groupe amino ou hydroxyalkyle en $C_1$ à $C_4$, et $R^5$ représente un groupe sulfonique, un groupe phosphonique ou un groupe carboxyle.

**7.** Structure absorbante selon l'une quelconque des revendications précédentes, **caractérisée en ce que** l'amidon qui est incorporé dans le superabsorbant, est de l'amidon de maïs naturel ou prégélatinisé, de l'amidon de maïs cireux naturel ou prégélatinisé, de l'amidon de pomme de terre naturel ou prégélatinisé, de l'amidon de blé naturel ou prégélatinisé, de l'amidon d'amylo-maïs naturel ou prégélatinisé, ou de l'amidon de tapioca naturel ou prégélatinisé, et/ou **en ce que** l'amidon chimiquement modifié qui est incorporé dans le superabsorbant, est de l'amidon dégradé par catalyse acide, par voie enzymatique ou par voie thermique, de l'amidon oxydé, un éther d'amidon comme l'allyl-amidon ou un hydroxyalkyl-amidon tel que le 2-hydroxyéthyl-amidon, le 2-hydroxypropyl-amidon ou le 2-hydroxy-3-triméthylammoniopropyl-amidon, ou un carboxyalkyl-amidon tel que le carboxyméthyl-amidon, un ester d'amidon comme le formiate d'amidon, l'acétate d'amidon, l'acrylate d'amidon, le méthacrylate d'amidon ou le benzoate d'amidon, un ester d'amidon comme le succinate d'amidon ou le maléate d'amidon, un ester carbamique de l'amidon (uréthane d'amidon), un ester dithiocarbonique de l'amidon (xanthate d'amidon), ou un ester de l'amidon avec un acide minéral, comme le sulfate d'amidon, le nitrate d'amidon ou le phosphate d'amidon, un ester d'éther d'amidon comme l'acétate de 2-hydroxyalkyl-amidon, ou des acétals totaux d'amidon, comme les produits de réaction de l'amidon avec des éthers vinyliques, aliphatiques ou cycliques.

**8.** Structure absorbante selon l'une quelconque des revendications précédentes, **caractérisée en ce que** l'amidon incorporé dans le superabsorbant, est de l'amidon de maïs prégélatinisé ou de l'amidon de pomme de terre prégélatinisé, et/ou **en ce que** l'amidon chimiquement modifié incorporé dans le superabsorbant est du carboxyméthyl-amidon, du succinate d'amidon ou du maléate d'amidon.

**9.** Structure absorbante selon l'une quelconque des revendications précédentes, **caractérisée en ce que** l'amorceur radicalaire employé pour la production du superabsorbant est un composé qui contient au moins trois groupes hydroperoxyde, peroxyde ou azo.

**10.** Structure absorbante selon l'une quelconque des revendications précédentes, **caractérisée en ce que** l'amorceur radicalaire employé pour la production du superabsorbant est un produit de réaction d'azobisbutyronitrile et de triméthylolpropane.

11. Structure absorbante selon l'une quelconque des revendications précédentes, **caractérisée en ce que** l'amorceur radicalaire employé pour la production du superabsorbant est un polyhydroperoxyde qui a été obtenu par oxydation anodique d'un acide polycarboxylique en présence d'oxygène.

12. Structure absorbante selon l'une quelconque des revendications précédentes, **caractérisée en ce que** l'on a produit le superabsorbant en polymérisant une solution contenant 15 à 60 % en poids d'un ou plusieurs monomères hydrophiles, en présence d'amidon et/ou d'amidon chimiquement modifié, au moyen d'un procédé de polymérisation en gel, en présence d'un amorceur radicalaire qui est capable de former des triradicaux ou des polyradicaux.

13. Produit absorbant (11) tel qu'une couche, une protection contre l'incontinence ou une serviette hygiénique, qui comprend un corps absorbant, entouré d'un matériau formant enveloppe, qui est au moins partiellement perméable aux liquides, **caractérisé en ce que** ledit corps absorbant comprend une structure absorbante selon l'une quelconque des revendications 1 à 12.

14. Produit absorbant selon la revendication 13, pour lequel le matériau formant enveloppe comprend un matériau de surface (12) perméable aux liquides, du côté de la structure absorbante (3) qui est destiné à faire face à un utilisateur lors de l'utilisation, et un matériau-barrière (13), imperméable aux liquides, du côté de la structure absorbante (3) qui est destiné à être à l'opposé d'un utilisateur lors de l'utilisation,
**caractérisé en ce que** le matériau de surface (12), perméable aux liquides, et/ou le matériau-barrière (13) comprennent une ou plusieurs matières biodégradables, facilement renouvelables, dans un mélange qui représente plus d'environ 10 % en poids du poids total du matériau formant enveloppe.

15. Produit absorbant selon la revendication 14, **caractérisé en ce que** de l'amidon est incorporé dans la matière biodégradable.

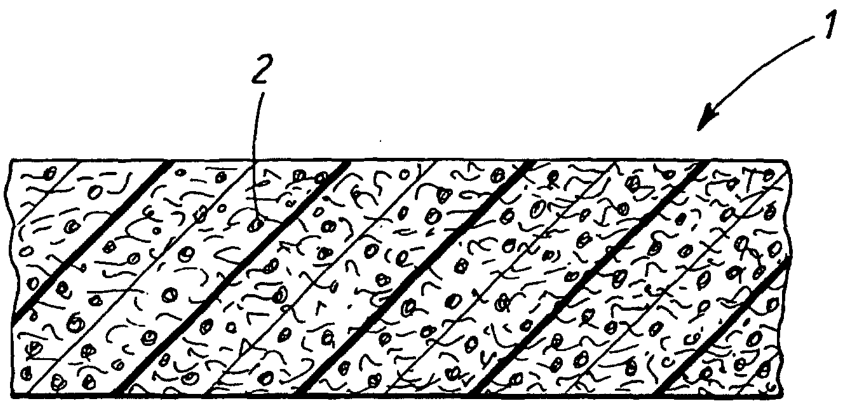


FIG.1

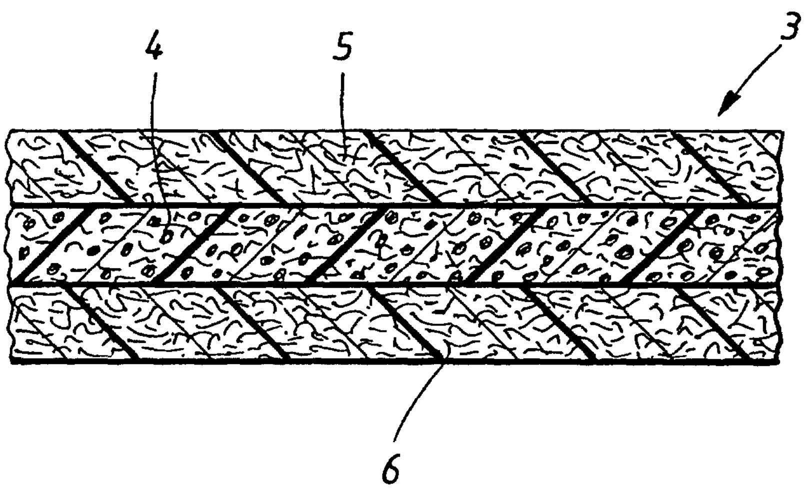


FIG.2

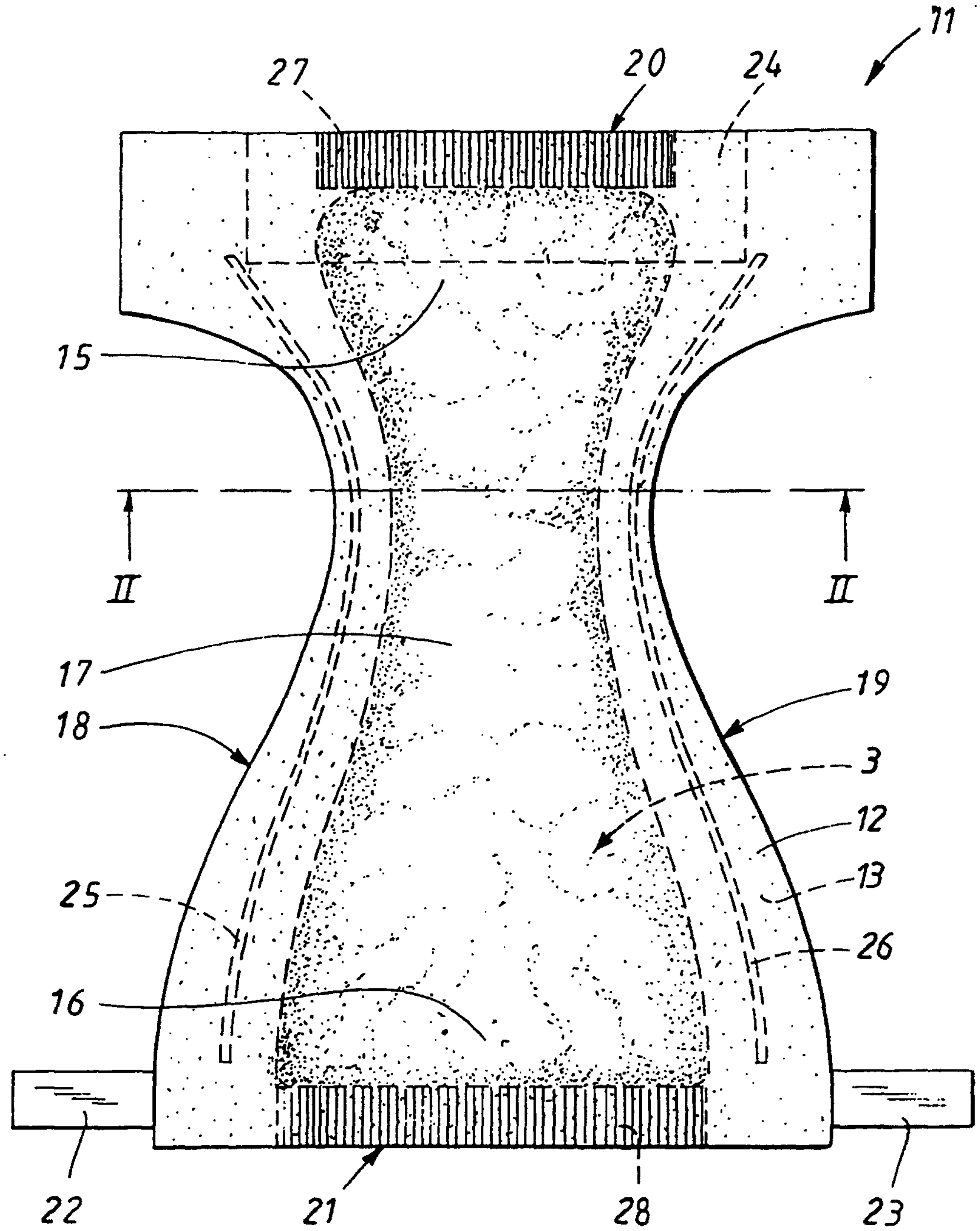
11
27
20
24
15
II
II
17
19
18
3
12
13
25
26
16
22
21
28
23

FIG. 3

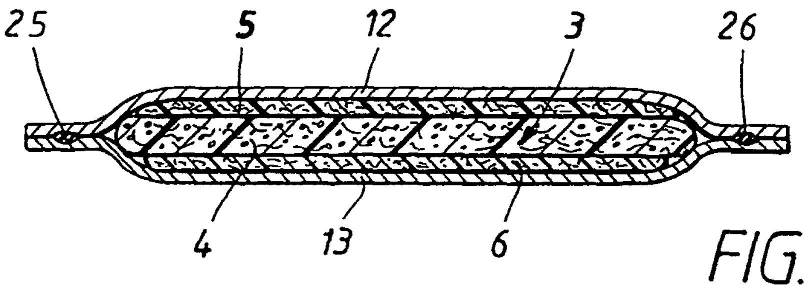
25
5
12
3
26
4
13
6

FIG. 4